# FASCICULE DE BREVET EUROPEEN

(11) **EP 0 850 034 B1**
(45) Date de publication et mention de la délivrance du brevet: **10.11.1999**
(21) Numéro de dépôt: 96931106.7
(22) Date de dépôt: 11.09.1996
(51) Int. Cl.: A61F 13/14

(54) **PANSEMENT POUR CICATRICES HYPERTROPHIQUES OU CHELOIDES DES SEINS**
VERBAND FÜR HYPERTROPHE ODER KELOIDALE BRUSTNARBEN
WOUND DRESSING FOR BREAST CHELOID OR HYPERTROPHIC SCARS

(30) Priorité: 12.09.1995 FR 9510656
(43) Date de publication de la demande: 01.07.1998
(73) Titulaire: Zagame, André, F-61110 Rémalard (FR)
(72) Inventeur: Zagame, André, F-61110 Rémalard (FR)
(74) Mandataire: Jaunez, Xavier
(86) Numéro de dépôt international: FR9601395
(87) Numéro de publication internationale: WO9709951

(56) Documents cités:
- WO-A-88/06877
- BE-A- 482 682
- FR-A- 2 505 620
- US-A- 2 869 553

## Description

La présente invention concerne un élément souple auto-adhésif à usage médical externe, pour le traitement des cicatrices hypertrophiques ou chéloïdes consécutives aux interventions mammaires.

L'état de la technique est illustré par le document WO-A-88 06877, qui décrit un patch médical adhésif destiné à protéger le tétin et la zone aréolaire du sein suite à une opération chirurgicale. Le patch est circulaire, et il est maintenu en place par quatre pattes adhésives disposées en croix. La zone concernée du sein est de facto très limitée.

Pour compléter l'état de la technique, on peut également citer d'autres dispositifs qui ne sont pas à usage médical, mais sont conçus pour assurer un maintien satisfaisant du sein. Il s'agit notamment de dispositifs du type soutien-gorge adhésif (voir par exemple les documents FR-A-2 505 620 et US-A-2,869,553), ou du type coque moulée destinée à servir de raidisseur implantable dans un soutien-gorge traditionnel (voir par exemple le document BE-A-482 682).

La chirurgie mammaire, qu'elle soit réparatrice ou reconstructive, implique la mise en oeuvre de plusieurs types de techniques chirurgicales, parmi lesquelles le chirurgien sélectionne celle qui est la mieux adaptée à la forme du sein. On peut citer la technique péri-aréolaire, qui laisse une cicatrice sensiblement circulaire dans la zone aréolaire du sein. Il y a aussi la technique en L ou en T, qui laisse une cicatrice formée d'une première ligne descendant de la zone péri-aréolaire du sein jusqu'au pli sous-mammaire, et d'une deuxième ligne (droite ou incurvée) s'étendant suivant le pli sous-mammaire, d'un côté ou de chaque côté de la première ligne. Cette technique en L ou en T peut d'ailleurs se combiner à la technique péri-aréolaire, la première ligne de la cicatrice étant alors surmontée d'une cicatrice sensiblement circulaire.

Le traitement de telles cicatrices dans un but d'esthétique est délicat, car le sein est un organe mou difficile à comprimer, surtout dans sa zone péri-aréolaire. De plus les patients sont naturellement peu enclins à utiliser un élément rigide ou semi-rigide ou une sangle enserrant partiellement ou totalement la poitrine pour exercer une pression localisée sur les zones cicatricielles.

On pourrait alors être tenté d'utiliser un élément souple du type de ceux que l'on utilise déjà pour le traitement des cicatrices dans une zone du corps présentant un appui osseux, mais on se heurte encore à plusieurs difficultés.

On connaît par exemple des éléments souples réalisés à partir d'un gel non-adhésif à base de silicone ou à base de copolymères tri-blocs, ces gels à usage médical étant habituellement conditionnés sous forme de plaques, de manchons, ou de bandes. Ces plaques, manchons, ou bandes présentent une souplesse avantageuse, mais leur épaisseur est importante (3 à 5 mm en général), ce qui nuit à l'esthétique, et ils nécessitent des moyens supplémentaires pour leur mise en place et leur maintien (bandes adhésives, etc...), de sorte qu'une application de tels éléments souples pour le traitement des cicatrices mammaires impliquerait des risques importants de déplacement indésirable induisant un mauvais positionnement par rapport à la poitrine du patient, et par suite une action thérapeutique amoindrie. De plus, et surtout dans le cas des manchons, ils exercent également une application sur une surface qui ne se limite pas à la zone cicatricielle à traiter, ce qui présente un inconvénient important en raison de l'effet occlusif du produit, et du risque de macération qui en découle. Par suite, un tel élément souple apparaît difficilement applicable pour le traitement de cicatrices consécutives aux interventions mammaires.

On connaît également des éléments souples constitués par un film à base de silicone dont une face est enduite d'un gel adhésif à base de silicone (voir par exemple le document US-A-4,991,574). Il s'agit en l'espèce de pansements destinés à être appliqués sur des plaies ouvertes ou sur des cicatrices, ces pansements étant découpés à partir d'une plaque rectangulaire en général de 10 cm x 15 cm. Cependant, ce type d'élément souple présente encore une épaisseur relativement importante (en général au moins 3 à 4 mm), et une souplesse très limitée, de sorte que le caractère traumatisant du port d'un tel élément est encore renforcé du fait de sa faible discrétion. La souplesse médiocre rend un élément de ce type difficilement adaptable à une conformation en surface gauche ou complexe, de sorte que son utilisation apparaît impropre pour le traitement de cicatrices consécutives à des interventions mammaires. L'épaisseur importante est aussi un facteur défavorable sur le plan de l'esthétique et du confort, ce qui est particulièrement sensible pour la zone de la poitrine. Enfin, ce produit est relativement fragile et sa durée de vie est limitée à une quinzaine de jours, ce qui en fait un produit coûteux.

L'invention a pour but de réaliser un élément souple auto-adhésif permettant un traitement efficace des cicatrices hypertrophiques ou chéloïdes consécutives aux interventions mammaires, et ne présentant pas les inconvénients des éléments souples précités.

L'invention a également pour but de réaliser un élément souple de type auto-adhésif qui soit efficace pour différents types de cicatrices mammaires dépendant du type d'intervention chirurgicale subie par le patient.

Il s'agit plus particulièrement d'un élément souple auto-adhésif à usage médical externe pour le traitement des cicatrices hypertrophiques ou chéloïdes consécutives aux interventions mammaires, caractérisé en ce qu'il présente un profil général en forme d'ancre marine, avec une branche centrale essentiellement rectiligne et deux branches latérales incurvées, la branche centrale étant agencée pour s'étendre jusqu'à la zone péri-aréolaire du sein concerné de façon à couvrir cette zone, et les deux branches latérales étant agencées pour s'étendre le long du pli sous-mammaire du sein concerné de façon à couvrir au moins partiellement ce pli, avec une première branche pour la partie du pli allant vers la zone sternale du patient et une deuxième branche pour la partie du pli allant vers la zone axillaire du patient.

Un tel élément souple présente ainsi une grande polyvalence au regard des techniques chirurgicales utilisées, qu'il s'agisse de techniques péri-aréolaires et/ou de techniques en L ou en T. Il devient dès lors possible de recommander systématiquement l'utilisation d'un tel élément souple auto-adhésif après une intervention chirurgicale mammaire, sans avoir à procéder à une découpe appropriée à; la circonstance.

De préférence, la branche centrale se termine par une partie d'extrémité arrondie recouvrant toute la zone aréolaire du sein concerné, et a une largeur essentiellement constante qui est la même que celle de sa partie d'extrémité arrondie. On est ainsi assuré de pouvoir recouvrir convenablement à la fois une cicatrice consécutive à une technique de chirurgie péri-aréolaire et les lignes de cicatrice consécutives à une technique chirurgicale en L ou en T descendant de la zone péri-aréolaire du sein jusqu'au pli sous-mammaire.

Avantageusement, la première branche latérale est plus courte que la deuxième branche latérale, cette dernière s'étendant jusqu'à la zone axillaire du patient. En particulier, les branches latérales ont une largeur essentiellement constante et se terminent par une partie d'extrémité arrondie. La géométrie particulière des branches latérales de l'élément souple assure ainsi un recouvrement optimal de la zone concernée du pli sous-mammaire pour tous les cas de cicatrices consécutives à une intervention réalisée conformément à une technique en L ou en T.

De préférence, l'élément souple est constitué par une couche de support sur laquelle est fixée une couche de gel adhésif dans la masse. En variante, on fixe sur une couche de support une couche de gel essentiellement non-adhésif dont la face libre est revêtue d'un film adhésif sur sa face externe.

Le matériau constituant la couche de support sera de préférence choisi dans le groupe constitué par les matériaux textiles, éventuellement élastiques, les matières plastiques, les papiers traités, et les gels souples essentiellement non-adhésifs.

Selon un premier mode d'application de l'invention, l'élément souple précité présente une face adhésive qui est destinée à être appliquée sur le corps du patient, cette face adhésive pouvant être revêtue d'un film de protection qui est enlevé juste avant la pose de l'élément souple.

Selon une variante d'application, l'élément souple présente une face adhésive qui est destinée à être appliquée contre la face interne d'un bonnet de soutiengorge dont l'élément souple auto-adhésif constitue alors une doublure.

D'autres caractéristiques et avantages de l'invention apparaîtront plus clairement à la lumière de la description qui va suivre et des dessins annexés, concernant des modes de réalisation particuliers, en référence aux figures où :
- la figure 1 illustre deux éléments souples auto-adhésifs conformes à l'invention, mis à plat, destinés à être appliqués respectivement sur un sein droit et un sein gauche;
- la figure 2 illustre une patiente portant pour chaque sein un élément souple en forme d'ancre marine conforme à la figure 1;
- la figure 3 est une autre vue en perspective illustrant une patiente portant un tel élément souple auto-adhésif pour son sein droit seulement;
- la figure 4 illustre en perspective un soutien-gorge de type conventionnel dont les deux bonnets sont garnis intérieurement d'un élément souple auto-adhésif, chaque élément souple formant ainsi une doublure du bonnet concerné ;
- les figures 5A, 5B, 5C, 5D, 5E, 5F sont des coupes selon V-V d'un élément souple auto-adhésif illustré à la figure 1, illustrant divers modes d'exécution de cet élément souple, avec des couches de support de différents matériaux, et des couches de gel qui est adhésif dans la masse ou essentiellement non-adhésif.

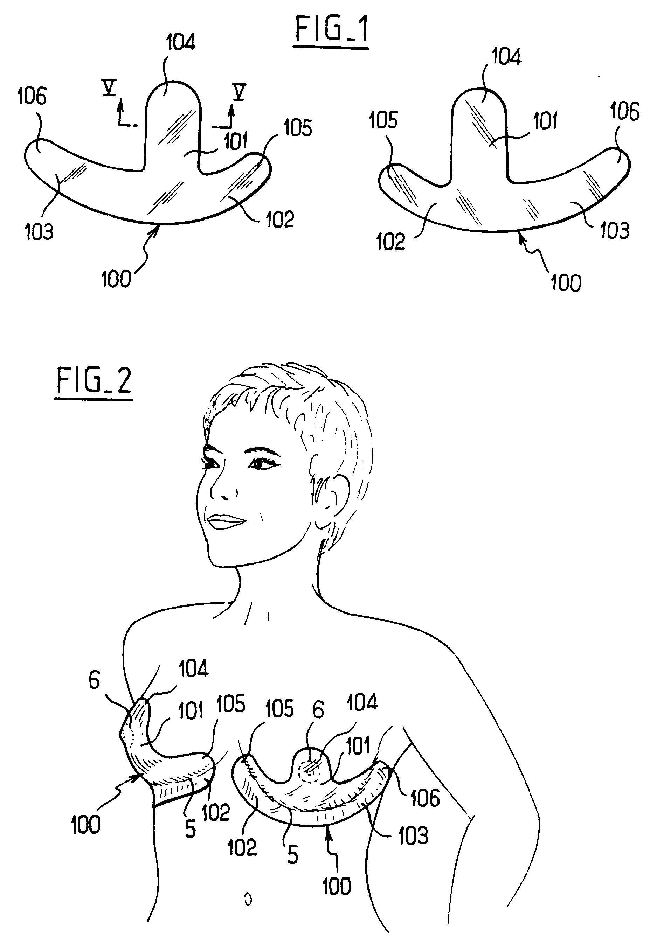

Sur la figure 1, on distingue deux éléments souples auto-adhésifs 100 conformes à l'invention, l'élément de gauche étant associé à la pose sur un sein droit, et l'élément de droite sur un sein gauche.Chaque élément souple 100 présente un profil général en forme d'ancre marine, avec une branche centrale essentiellement rectiligne 101 et deux branches latérales incurvées 102, 103. La branche centrale 101 se termine ici par une partie d'extrémité arrondie 104, et elle présente une largeur essentiellement constante qui est la même que celle de sa partie d'extrémité arrondie 104.

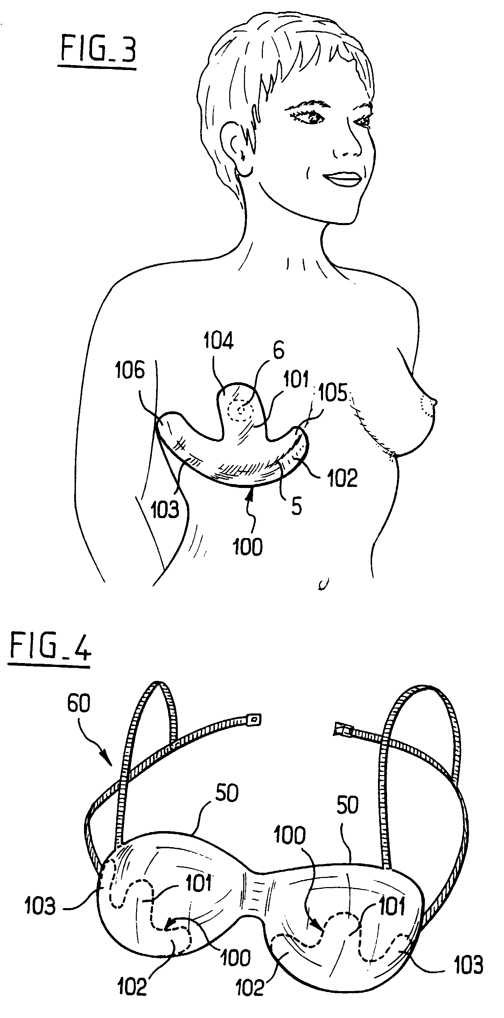

Ainsi que cela est mieux visible sur les figures 2 et 3, la branche centrale 101 de l'élément souple 100 est agencée pour s'étendre jusqu'à la zone péri-aréolaire du sein concerné de façon à couvrir cette zone, la partie d'extrémité arrondie 104 recouvrant en effet toute la zone aréolaire 6 du sein. Les deux branches latérales 102, 103 sont quant à elles agencées pour s'étendre le long du pli sous-mammaire 5 du sein concerné, de façon à couvrir au moins partiellement ce pli. Chaque élément souple 100 comporte ainsi une première branche 102 pour la partie du pli sous-mammaire allant vers la zone sternale du patient, et une deuxième branche 103 pour la partie dudit pli allant vers la zone axillaire du patient. En l'espèce, on constate que la première branche latérale 102 est plus courte que la deuxième branche latérale 103, cette dernière s'étendant juste à la zone axillaire du patient. Cette asymétrie des éléments souples constitue peut-être un léger inconvénient dans la mesure où l'on est obligé de réaliser et de stocker des éléments gauche et droit, mais ce léger inconvénient est largement compensé par l'avantage d'une adaptation aisée et très fidèle de chaque élément souple à la morphologie du patient. En outre, la branche latérale plus longue s'étendant jusqu'à la zone axillaire du patient permet d'assurer un maintien parfait de l'élément souple par rapport à une ligne verticale descendant depuis la zone aréolaire du sein, de sorte que cette zone, ainsi que la zone qui la raccorde au pli sous-mammaire, reste toujours correctement couverte par l'élément souple, en dépit des mouvements du patient. On observera en outre que les branches latérales 102, 103 ont ici une largeur essentiellement constante, et se terminent par une partie d'extrémité arrondie respectivement 105, 106. Dans la pratique, on choisira une largeur suffisante pour être assuré d'un recouvrement correct de toute la partie concernée du pli sous-mammaire 5.

Les figures 2 et 3 illustrent un premier mode d'utilisation de l'élément souple 100 conforme à l'invention, selon lequel la face adhésive de l'élément souple est appliquée directement sur le corps du patient. Dans ce cas, chaque élément souple tient par lui-même, sans qu'il soit nécessaire de prévoir un quelconque moyen extérieur de maintien. En outre, dans la mesure où la constitution de l'élément souple est choisie pour avoir une faible épaisseur, cet élément souple est extrêmement discret, et permet à la personne de porter son soutien-gorge habituel.

Selon un autre mode d'utilisation de l'invention, la face adhésive de l'élément souple 100 est destinée à être appliquée contre la face interne d'un bonnet de soutien-gorge dont l'élément souple auto-adhésif constitue alors une doublure. Une telle variante est illustrée sur la figure 4, où l'on distingue un soutien-gorge de type conventionnel 60, dont l'un au moins des deux bonnets 50 (ici les deux) est garni intérieurement d'un élément souple auto-adhésif 100 conforme à ceux précédemment décrits. Dans ce cas, le ou chaque bonnet qui est garni d'un élément souple correctement positionné va permettre un positionnement naturel et automatique du ou des deux éléments souples utilisés dès que le soutien-gorge est mis en place. Un tel mode d'application est avantageux dans la mesure où il permet au patient d'utiliser son soutien-gorge habituel, avec une discrétion qui parvient à faire oublier le port d'un élément thérapeutique qui intervient dès que le soutien-gorge est mis en place.

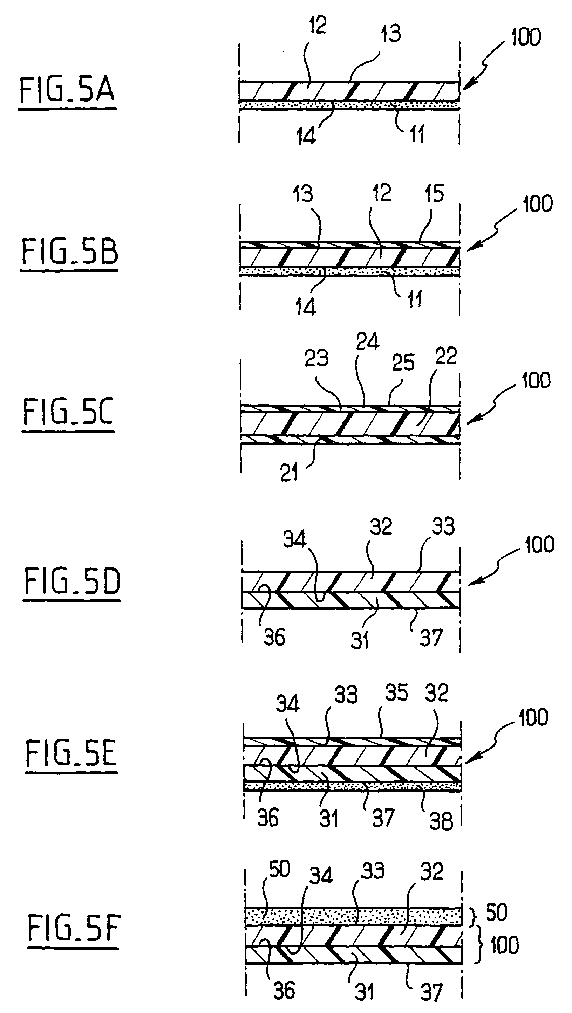

On va maintenant décrire différents exemples de constitution d'éléments souples auto-adhésifs 100 en couches superposées, en référence aux figures 5A à 5F.

Sur la figure 5A, l'élément souple auto-adhésif 100 est constitué par une couche de support 11 réalisée en matériau textile, sur laquelle est fixée une couche 12 de gel à usage médical externe, ce gel étant adhésif dans la masse. Dans ce cas, la face libre, notée 13, de la couche 12 de gel adhésif est adhésive. Ainsi que cela est illustré sur la figure 5B, il sera dans la pratique commode de prévoir que la face libre 13 de la couche 12 de gel adhésif soit revêtue d'un film de protection 15, par exemple en matière plastique, lequel film est enlevé juste avant la pose de l'élément souple auto-adhésif 100. Il pourra s'agir d'un simple film pelable analogue à ceux que l'on trouve sur de nombreux produits auto-adhésifs du domaine médical. La présence d'un tel film de protection 15 est surtout intéressante lorsque la face libre de la couche de gel adhésif est destinée à être appliquée directement sur le corps du patient. Le matériau textile constituant la couche de support 11 peut être un tissu, et éventuellement un tissu élastique, de façon à obtenir dans ce cas un élément souple qui est de surcroît compressif.

Bien que plusieurs gels adhésifs soient envisageables pour constituer la couche 12 de gel adhésif dans la masse, on va indiquer ci-après une composition particulière de gel adhésif qui est apparue particulièrement satisfaisante pour réaliser l'élément souple auto-adhésif de l'invention.

Conformément à cette composition particulière, le gel adhésif de la couche 12 est réalisé à partir d'au moins un copolymère bloc à base de styrène et d'isoprène dans une huile minérale pharmaceutique formant solvant, et d'une résine d'hydrocarbures rentrant dans ce gel dans une proportion prédéterminée qui est choisie pour conférer au gel l'adhésivité désirée. Le choix particulier du ou des copolymères précités permet d'obtenir un gel plus ou moins mou, se prêtant particulièrement bien à une conformation tridimensionnelle. Par ailleurs, le choix de la proportion de résine d'hydrocarbures dans le gel adhésif permet d'obtenir l'adhésivité désirée convenant idéalement à l'application particulière concernée. De préférence, on utilisera un gel réalisé à partir d'au moins un copolymère bloc linéaire à base de styrène et d'isoprène comportant environ 15% en poids de styrène, ce polymère ou ce mélange de copolymères rentrant dans le gel adhésif dans une proportion en poids essentiellement comprise entre 5% et 40%. A titre d'exemple, on peut citer tout particulièrement le copolymère bloc linéaire à base de styrène et d'isoprène commercialisé sous la dénomination KRATON D-KX 601 CS par la société SHELL CHEMICAL COMPANY (KRATON® est une marque déposée par la société SHELL). On utilisera alors, de préférence, une huile paraffinique légère, telle que celle qui est commercialisée sous la marque ONDINA 15® par la société des pétroles SHELL. Pour la résine, on pourra utiliser la résine commercialisée sous la dénomination REGALITE R 101 par la société HERCULES S.A. (REGALITE ® est une marque déposée).

La figure 5C illustre un autre élément souple auto-adhésif 100 conforme à l'invention, dont la couche de support 21 est un film de matière plastique, ou une feuille de papier traité (par exemple un papier sulfurisé). Par ailleurs, la couche de gel 22 est ici essentiellement non-adhésive, de sorte qu'il est alors nécessaire de prévoir de rapporter sur la face libre 23 de cette couche, un film 24 qui est adhésif sur sa face externe 25. Pour le gel non-adhésif, on pourra utiliser un gel à base de silicone ou à base de copolymères tri-blocs. On peut par exemple citer les gels à base de copolymères blocs styrène-éthylène/butylène-styrène dans une huile minérale. On peut également citer les gels à base de copolymères blocs styrène-éthylène/butylène-styrène réalisés avec des KRATON® G 1650, 1651, 1652, 1654, (KRATON® est une marque déposée par la société SHELL).

La figure 5D illustre une autre variante dans laquelle la couche de support 31 est réalisée en un gel souple essentiellement non-adhésif, par exemple un gel du type précité à base de copolymères blocs styrène-éthylène/butylène-styrène dans une huile minérale légère. Une couche 32 de gel adhésif dans la masse est alors fixée sur cette couche de support 31. On pourra utiliser pour cette dernière couche la composition particulière précitée déjà indiquée pour le gel adhésif dans la masse de la figure 5A. Dans ce cas, la face libre 37 de l'élément souple 100 est non-adhésive, tandis que l'autre face libre 33 est adhésive. Pour le gel non-adhésif, on pourra par exemple utiliser un gel tel que celui qui est commercialisé sous la dénomination KRATON G 1654 X par la société SHELL CHIMIE ((KRATON® est une marque déposée par la société SHELL). En variante on pourra utiliser un autre KRATON G commercialisé par la même société, tel que le KRATON G -1651 qui diffère du précédent par sa proportion de styrène.

La figure 5E illustre une variante de la figure précédente, dans laquelle la face libre 33 de la couche 32 de gel adhésif dans la masse est revêtue d'un film de protection 35 qui est enlevé juste avant la pose de l'élément souple auto-adhésif, et la face externe 37 de la couche de support 31 est revêtue d'une couche de protection 38 en tissu visant à éviter de tacher de gras un vêtement venant au contact de la surface extérieure de l'élément souple.

La figure 5F illustre une autre variante de la figure 5D, selon laquelle la face externe 37 de la couche de support 31 en gel souple essentiellement non-adhésif est destinée à être appliquée sur la zone concernée du corps du patient, tandis que la face libre 33 de la couche 32 de gel adhésif est appliquée contre la face interne du bonnet 50 d'un soutien-gorge, dont l'élément souple auto-adhésif constitue alors une doublure, conformément à ce qui a déjà été décrit en référence à la figure 4.

On est ainsi parvenu à réaliser un élément souple auto-adhésif qui est particulièrement performant pour le traitement des cicatrices mammaires, et ce quel que soit le type de technique chirurgicale utilisé lors de l'intervention concernée. Dans tous les cas, on est assuré d'une application intime sur la peau du patient, et donc d'un effet thérapeutique optimal pour le traitement des cicatrices. En outre, cet élément souple auto-adhésif est discret et confortable à porter grâce à sa faible épaisseur et sa grande souplesse, dans la mesure où l'on choisit des éléments constitutifs favorables tels que ceux qui ont été indiqués plus haut à titre d'exemple.

L'invention n'est pas limitée aux modes de réalisation qui viennent d'être décrits, mais englobe au contraire toute variante reprenant, avec des moyens équivalents, les caractéristiques essentielles énoncées plus haut.

## Revendications

1. Elément souple auto-adhésif à usage médical externe (100) pour le traitement des cicatrices hypertrophiques ou chéloïdes consécutives aux interventions mammaires, caractérisé en ce qu'il présente un profil général en forme d'ancre marine, avec une branche centrale essentiellement rectiligne (101) et deux branches latérales incurvées (102, 103), la branche centrale (101) étant agencée pour s'étendre jusqu'à la zone péri-aréolaire du sein concerné de façon à couvrir cette zone, et les deux branches latérales (102, 103) étant agencées pour s'étendre le long du pli sous-mammaire du sein concerné de façon à couvrir au moins partiellement ce pli, avec une première branche (102) pour la partie du pli allant vers la zone sternale du patient et une deuxième branche (103) pour la partie du pli allant vers la zone axillaire du patient.

2. Elément souple selon la revendication 1, caractérisé en ce que la branche centrale (101) se termine par une partie d'extrémité arrondie (104) recouvrant toute la zone aréolaire du sein concerné.

3. Elément souple selon la revendication 2, caractérisé en ce que la branche centrale (101) a une largeur essentiellement constante qui est la même que celle de la partie d'extrémité arrondie (104).

4. Elément souple selon l'une des revendications 1 à 3, caractérisé en ce que la première branche latérale (102) est plus courte que la deuxième branche latérale (103), cette dernière s'étendant jusqu'à la zone axillaire du patient.

5. Elément souple selon la revendication 4, caractérisé en ce que les branches latérales (102 ; 103) ont une largeur essentiellement constante et se terminent par une partie d'extrémité arrondie (105 ; 106).

6. Elément souple selon l'une des revendications 1 à 5, caractérisé en ce qu'il est constitué par une couche de support (11, 31) sur laquelle est fixée une couche (12, 32) de gel adhésif dans la masse.

7. Elément souple selon l'une des revendications 1 à 5, caractérisé en ce qu'il est constitué par une couche de support (21) sur laquelle est fixée une couche (22) de gel essentiellement non-adhésif dont la face libre est revêtue d'un film (24) adhésif sur sa face externe.

8. Elément souple selon la revendication 6 ou la revendication 7, caractérisé en ce que le matériau constituant la couche de support (11, 21, 31) est choisi dans le groupe constitué par les matériaux textiles, éventuellement élastiques, les matières plastiques, les papiers traités, et les gels souples essentiellement non-adhésifs.

9. Elément souple selon l'une des revendications 6 à 8, caractérisé en ce qu'il présente une face adhésive (13 ; 25 ; 33) qui est destinée à être appliquée sur le corps du patient, ladite face adhésive pouvant être revêtue d'un film de protection (15 ; 35) qui est enlevé juste avant la pose de l'élément souple.

10. Elément souple selon l'une des revendications 6 à 8, caractérisé en ce qu'il présente une face adhésive (13 ; 25 ; 33) qui est destinée à être appliquée contre la face interne d'un bonnet de soutien-gorge dont l'élément souple auto-adhésif (100) constitue alors une doublure.

## Claims

1. A flexible adhesive element (100) for external medical use in the treatment of hypertrophic or cheloid scars following breast surgery, the element being characterized in that its profile is generally anchor-shaped, with an essentially rectilinear central branch (101) and two curved side branches (102, 103), the central branch (101) being designed to extend to the peri-areolar zone of the breast concerned so as to cover said zone, and the two side branches (102, 103) being designed to extend along the fold beneath the breast concerned so as to cover said fold at least in part, with a first branch (102) being for the portion of the fold that goes towards the sternum zone of the patient, and a second branch (103) being for the portion of the fold that goes towards the axillary zone of the patient.

2. A flexible element according to claim 1, characterized in that the central branch (101) is terminated in a rounded end portion (104) covering all of the areolar zone of the breast concerned.

3. A flexible element according to claim 2, characterized in that the central branch (101) has a width that is essentially constant and is the same as the width of the rounded end portion (104).

4. A flexible element according to any one of claims 1 to 3, characterized in that the first side branch (102) is shorter than the second side branch (103), the second side branch extending to the axillary zone of the patient.

5. A flexible element according to claim 4, characterized in that the side branches (102; 103) are of essentially constant width and terminate in respective rounded end portions (105; 106).

6. A flexible element according to any one of claims 1 to 5, characterized in that it is constituted by a support layer (11, 31) on which there is fixed a layer (12, 32) of gel that is adhesive throughout.

7. A flexible element according to any one of claims 1 to 5, characterized in that it is constituted by a support layer (21) on which there is fixed a layer (22) of gel that is essentially non-adhesive, with the free face thereof being covered in a film (24) that is adhesive on its outside face.

8. A flexible element according to claim 6 or claim 7, characterized in that the material constituting the support layer (11, 21, 31) is selected from the group constituted by textile materials, optionally elastic textile materials, plastics materials, treated papers, and essentially non-adhesive flexible gels.

9. A flexible element according to any one of claims 6 to 8, characterized in that it has an adhesive face (13; 25; 33) designed to be applied against the body of the patient, said adhesive face being optionally covered in a protective film (15; 35) which is removed immediately before the flexible element is put into place.

10. A flexible element according to any one of claims 6 to 8, characterized in that it has an adhesive face (13; 25; 33) which is designed to be applied to the inside face of a bra cup for which the flexible adhesive element (100) then constitutes a lining.

## Patentansprüche

1. Selbstklebendes weiches Element (100) zur äußeren medizinischen Anwendung für die Behandlung von hypertrophen oder keloidalen Narben als Folge von Brustoperationen, dadurch **gekennzeichnet**, daß es ein Gesamtprofil in Form eines Schiffsankers aufweist, mit einem im wesentlichen geradlinigen zentralen Arm (101) und zwei gebogenen seitlichen Armen (102, 103), wobei der zentrale Arm (101) so angeordnet ist, daß er sich derart bis an den Umfangsbereich des Warzenhofes der betroffenen Brust erstreckt, daß er diesen Bereich überdeckt, und die beiden seitlichen Arme (102, 103) so angeordnet sind, daß sie sich derart entlang der unterhalb der Brust befindlichen Falte der betroffenen Brust erstrecken, daß sie diese Falte wenigstens teilweise überdecken, mit einem ersten Arm (102) für den Teil der Falte, der in Richtung des Brustbeinbereiches des Patienten geht, und einem zweiten Arm (103) für den Teil der Falte, der in Richtung des Achselbereichs des Patienten geht.

2. Weiches Element nach Anspruch 1, dadurch **gekennzeichnet**, daß der zentrale Arm (101) mit einem abgerundeten Endabschnitt (104) endet, der den gesamten Bereich des Warzenhofes der betroffenen Brust überdeckt.

3. Weiches Element nach Anspruch 2, dadurch **gekennzeichnet**, daß der zentrale Arm (101) eine im wesentliche konstante Breite hat, die gleich der des abgerundeten Endabschnittes (104) ist.

4. Weiches Element nach einem der Ansprüche 1 bis 3, dadurch **gekennzeichnet**, daß der erste seitliche Arm (102) kürzer als der zweite seitliche Arm (103) ist, wobei sich der letztere bis zum Achselbereich des Patienten erstreckt.

5. Weiches Element nach Anspruch 4, dadurch **gekennzeichnet**, daß die seitlichen Arme (102; 103) eine im wesentlichen konstante Breite haben und mit einem abgerundeten Endabschnitt (105; 106) enden.

6. Weiches Element nach einem der Ansprüche 1 bis 5, dadurch **gekennzeichnet**, daß es aus einer Stützschicht (11, 31) gebildet ist, auf der eine Schicht (12, 32) aus einem als Ganzes klebenden Gel aufgebracht ist.

7. Weiches Element nach einem der Ansprüche 1 bis 5, dadurch **gekennzeichnet**, daß es aus einer Stützschicht (21) gebildet ist, auf der eine Schicht (22) aus im wesentlichen nicht klebendem Gel aufgebracht ist, deren freie Seite von einem auf seiner Außenseite klebenden Film (24) überzogen ist.

8. Weiches Element nach Anspruch 6 oder Anspruch 7, dadurch **gekennzeichnet**, daß das die Stützschicht (11, 21, 31) bildende Material aus der Gruppe ausgewählt ist, die aus Textilstoffen, gegebenenfalls elastischen Textilstoffen, Kunststoffen, behandelten Papieren und weichen im wesentlichen nicht klebenden Gelen besteht.

9. Weiches Element nach einem der Ansprüche 6 bis 8, dadurch **gekennzeichnet**, daß es eine klebende Seite (13; 25; 33) aufweist, die dazu bestimmt ist, auf dem Körper des Patienten aufgelegt zu werden, wobei die klebende Seite von einem Schutzfilm (15; 35) überzogen sein kann, der kurz vor dem Anbringen des weichen Elementes entfernt wird.

10. Weiches Element nach einem der Ansprüche 6 bis 8, dadurch **gekennzeichnet**, daß es eine klebende Seite (13; 25; 33) aufweist, die dazu bestimmt ist, an der Innenseite eines Körbchens eines Büstenhalters angebracht zu werden, wobei das selbstklebende weiche Element (100) dann dessen Futter bildet.
